# EUROPÄISCHE PATENTSCHRIFT

(11) **EP 3 749 267 B1**
(45) Veröffentlichungstag und Bekanntmachung des Hinweises auf die Patenterteilung: **15.07.2026**
(21) Anmeldenummer: 19707272.1
(22) Anmeldetag: 07.02.2019
(51) Int. Cl.: A61F 11/20

(54) **OHRKATHETER**
EAR CATHETER
CATHÉTER D'OREILLE

(30) Priorität: 09.02.2018 DE 102018102937
(43) Veröffentlichungstag der Anmeldung: 16.12.2020
(73) Patentinhaber: Auriventis Medical GmbH, 79540 Lörrach (DE)
(72) Erfinder: SUDHOFF Holger, 33604 Bielefeld (DE)
(74) Vertreter: Schneiders & Behrendt Bochum
(86) Internationale Anmeldenummer: PCT/EP2019/052987
(87) Internationale Veröffentlichungsnummer: WO 2019/154902

(56) Entgegenhaltungen:
- US-A1- 2006 106 361
- US-A1- 2010 198 191
- US-A1- 2013 274 715

## Beschreibung

Die Erfindung betrifft einen Ohrkatheter zur nicht-invasiven Einführung in die Ohrtrompete, der es erlaubt, im Mittelohrbereich eine medikamentöse Behandlung über eine längere Zeit durchzuführen.

Es sind zahlreiche Erkrankungen des Mittel- und Innenohrs bekannt, die eine medikamentöse Behandlung erfordern. Soweit eine topische Behandlung durchgeführt werden muss, kann diese zum einen durch das Trommelfell und zum anderen über die Ohrtrompete durchgeführt werden. Auch die Behandlung des Innenohrs kann über den Mittelohrbereich erfolgen. Die Verabreichung von Medikamenten in flüssiger Form in die Ohrtrompete oder das Mittelohr ist aber in der Regel nicht nachhaltig, da das Medikament durch die Ohrtrompete in den Nasenrachenraum abläuft.

Eine Reihe von Infektionen, Entzündungen aber auch die Behandlung eines Hörsturzes oder der Meniére-Krankheit erfordert eine längerfristige Behandlung, die sich über Tage oder wenige Wochen hinziehen kann. In der Regel wird in diesem Fall ständig neues Medikament, etwa ein Corticosteroid, zugeführt. Diese Behandlung ist zeitaufwendig und verlangt eine regelmäßige und dauerhafte ärztliche Überwachung und Betreuung.

Aus diesem Grunde wäre es wünschenswert, über eine Vorrichtung zu verfügen, mit der ein Medikament in das Mittelohr appliziert und dort über längere Zeit vorrätig gehalten werden kann. Eine solche Applikation soll nach Möglichkeit nicht-invasiv erfolgen, also ohne Verletzung des Trommelfells. In diesem Fall kommt lediglich der natürliche Zugang über den Nasenrachenraum und die Ohrtrompete (die eustachische Röhre) in Frage.

Diese Aufgabe wird mit Hilfe eines Ohrkatheters der eingangs genannten Art gelöst, der einen Katheterschlauch, einen expandierbaren Ballon zum Verschluss der Ohrtrompete und einen Injektionskanal zur Einbringung von Medikamenten in den Mittelohrbereich verfügt.

Die Druckschrift US 2006/0106361 offenbart einen nicht-invasiven Ohrkatheter zur Einführung in die Ohrtrompete mit einem Katheterschlauch, einem expandierbaren Ballon zum Verschluss der Ohrtrompete und einem Injektionskanal zur Einführung eines Medikaments in den Mittelohrbereich.

Ein Ohrkatheter gemäß der vorliegenden Erfindung ist in Anspruch 1 definiert.

Ein solcher Ohrkatheter kann nicht-invasiv durch die Nase und den Nasenrachenraum in die Ohrtrompete eingeführt werden und dort so platziert werden, dass der Ballon nach Expansion die Ohrtrompete verschließt. Dieser Verschluss unterbricht die Verbindung des distal vom Ballon gelegenen Mittelohrbereichs zum Nasenrachenraum. Distal bedeutet in diesem Zusammenhang den zum Gehör und Mittelohr hinweisenden Teil der Ohrkatheters, während proximal den vom Gehör und Mittelohr wegweisenden Teil bezeichnet.

Bei dem erfindungsgemäßen Ohrkatheter handelt es sich um einen Ballonkatheter, der im Prinzip aufgebaut ist, wie ein Ballonkatheter zur Gefäßerweiterung, jedoch nicht der Erweiterung der Ohrtrompete dient sondern dem Verschluss. Insoweit wird der Ballon des Ohrkatheters nur mit einem minimalen Überdruck beaufschlagt dergestalt, dass sich eine Manschette ausbildet, die die Ohrtrompete verschließt (Low-Pressure-Cuff). Hierzu ist ein einziger Ballon ausreichend Der Schlauch des Ohrkatheters hat in etwa eine Länge von 15 cm, d. h. die Länge reicht aus, um den Katheter durch ein Nasenloch und den Nasenrachenraum in die Ohrtrompete einzuführen, wobei das proximale Ende im Wangenbereich des Patienten verbleibt.

Der Injektionskanal verläuft im Wesentlichen im Inneren des Ohrkatheters, abgesehen von seinem proximalen Ende, das seitlich aus dem Ohrkatheter herausragt. Ohrkatheter und Injektionskanal sind mit Anschlüssen für übliche Spritzen versehen, die das Befüllen des Ohrkatheters mit Druckmedium und das Einführen von Medikamentenlösung in das Innenohr erlauben. Bei den Anschlüssen handelt es sich zweckmäßigerweise um das in der Medizin übliche Luer-Lock-System, an die mit entsprechenden Einrichtungen verschiedene Spritzen angekuppelt werden können.

Für Ballon, Katheter und Injektionskanal können übliche Materialien verwandt werden, wie sie in der Ballonkathetertechnik üblich sind. Es handelt sich dabei um Kunststoffe, die medizinisch zugelassen sind, beispielsweise PVC, Polyethylen, Polypropylen und dergleichen. Die Materialien sollten hinreichend elastisch sein, um das Einführen in die Ohrtrompete zu erlauben.

Es ist vorteilhaft, den Katheter mit einer Handhabungshilfe für die Steuerung auszustatten. Des Weiteren ist es vorteilhaft, am Katheter ein gekrümmtes distales Ende vorzusehen, das die Steuerung und Positionierung des Ballons und Katheterendes in die Ohrtrompete hinein und in der Ohrtrompete erleichtert. Ein solches gekrümmtes Ende wird bei Kathetern und Führungsdrähten als "Pig-Tail" bezeichnet und ist weithin üblich.

Es versteht sich, dass der Ohrkatheter eine Verschlussvorrichtung aufweist, die das applizierte Druckmedium nach Befüllen zurückhält und den expandierten Zustand gewährleistet. Desgleichen weist der Injektionskanal entweder an seinem distalen oder an seinem proximalen Ende einen Verschluss auf, der das Abfließen des injizierten Medikaments durch den Injektionskanal verhindert. Es ist auch möglich, den Injektionskanal, der aus einem weichen Kunststoffschlauch bestehen kann, mit Hilfe des aufgeblasenen Ballons, der die Ohrtrompete verschließt, abzuquetschen.

Der erfindungsgemäße Ohrkatheter wird nicht-invasiv durch die Nase und den Nasenrachenraum in die Ohrtrompete eingeführt. Die Einführung kann ohrmikroskopisch überwacht werden. Sobald der expandierbare Ballon den zu verschließenden Teil der Ohrtrompete erreicht hat, wird er auf bekannte Art und Weise mit einer Flüssigkeit beaufschlagt und expandiert. Dabei ist es zweckmäßig, den Ballon als Low-Pressure-Cuff auszubilden und einen Druckbegrenzer vorzusehen, der zweckmäßigerweise den Druck auf ein Maximum von 1 bar (Überdruck) begrenzt. Zur Expansion des Ballons wird eine Flüssigkeit verwandt, vorzugsweise physiologische Kochsalzlösung. Zur Einbringung der Kochsalzlösung ist der proximale Anschluss des Ohrkatheters mit einem herkömmlichen Luer-Lock versehen, an den eine Spritze für die Kochsalzlösung angekoppelt werden kann. Der Katheterschlauch ist mit einem Einwegventil ausgestaltet, damit der Ballon den Druck hält.

Der Injektionskanal, in der Regel ein Injektionsschlauch, führt durch den Katheterschlauch und den Ballon in den Mittelohrbereich. Am proximalen Ende weist er ebenfalls einen Luer-Lock-Anschluss für eine Spritze auf über die das Medikament in den Mittelohrbereich eingespritzt werden kann. Am distalen Ende befindet sich ein Auslass, der atraumatisch ausgestaltet sein kann und mit einem Einwegventil versehen sein kann. Bei einem Einwegventil öffnet die Austrittsöffnung unter dem Druck der injizierten Lösung. Ist der Schlauch aus einem weichen, elastischen Material gefertigt, kann aber auch der Druck des Ballons zum Verschluss ausreichen. In diesem Fall ist der Injektionsschlauch unter dem Druck des Ballons "abgequetscht" und öffnet nur unter dem Druck der Injektionsflüssigkeit, wenn diese mit der Spritze eingeführt wird. Ein Einwegventil kann alternativ zur distalen Anordnung auch proximal angeordnet sein.

Der erfindungsgemäße Ohrkatheter ist besonders geeignet zur Einführung von Antibiotikalösungen in den Mittelohrbereich, was bei hartnäckigen bakteriellen Infektionen sinnvoll ist. Auch kann die Standardbehandlung eines Hörsturzes, die mit einem Corticosteroid erfolgt, sinnvoll über einen solchen Ohrkatheter durchgeführt werden. Die Standardtherapie verlangt, dass ein solches Corticosteroid über längere Zeit appliziert wird, in der Regel etwa zwei Wochen, wobei die Applikation täglich neu vorgenommen werden muss, da das Medikament in den Nasenrachenraum hinein abläuft. Üblich ist eine Behandlungsdauer von 14 Tagen. Der erfindungsgemäße Ohrkatheter ermöglicht eine sehr viel intensivere und kürzere Behandlung, da er die ständige Präsenz des Medikaments gewährleistet. Die Behandlungsdauer verkürzt sich dadurch erheblich, unter Umständen auf wenige Tage. Er ist insbesondere geeignet, das Medikament über eine längere Zeit, insbesondere auch mehrere Tage, im Mittelohr zurückzuhalten.

Ferner können auch entzündungshemmende Medikamente über den Ohrkatheter eingeführt werden.

Der Ohrkatheter wird über eine der Nasenöffnungen gelegt und nach Druckbeaufschlagung und Infusion des Medikaments mit einem geeigneten Pflaster auf der Wange festgelegt. Er verbleibt während der Behandlung in der Ohrtrompete. Der Patient bleibt mobil, ein längerer Aufenthalt in einer Klinik ist nicht erforderlich, eine regelmäßige ärztliche Überwachung reicht aus. Zur Überwachung kann die Ohrmikroskopie angewendet werden.

Nach der Behandlung kann der Ohrkatheter nach Druckentlastung ohne weiteres entfernt werden.

Der Ohrkatheter erlaubt auch die Behandlung von Erkrankungen des Innenohrs, da die Medikamente in der Regel durch die Membran des runden Fensters zum Innenohr durchtreten können. Es handelt sich dabei um einen Diffusionsprozess.

Die Erfindung wird durch die beiliegende Abbildung näher erläutert.

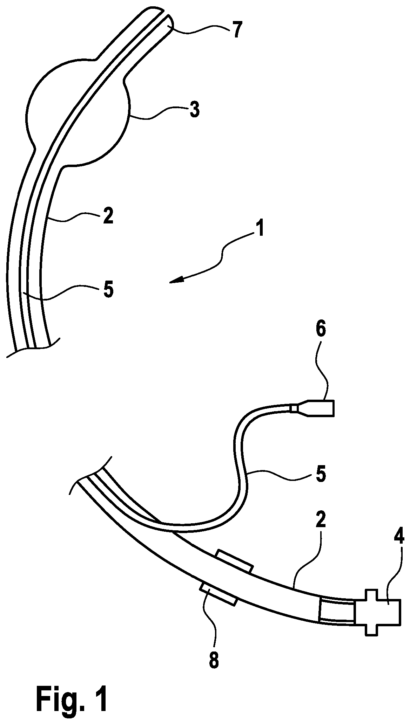

Der erfindungsgemäße Ohrkatheter 1 gemäß Figur 1 besteht aus einem Katheterschlauch 2, der in einem expandierbaren Ballon 3 (Cuff) distal endet. Proximal befindet sich ein Luer-Lock-Anschluss 4 für eine herkömmliche Spritze, mit der physiologische Kochsalzlösung zur Expansion des Ballons 3 eingeführt werden kann. Ein zwischengeschaltetes Manometer (nicht dargestellt) überwacht den Druck und begrenzt diesen auf 1 bar.

Im Zentrum des Katheters verläuft ein Injektionsschlauch 5, ebenfalls mit einem Luer-Lock-Anschluss 6, über den mittels einer Spritze Medikamentenlösung eingespritzt werden kann. Der Injektionskanal 5 endet in einer atraumatisch ausgebildeten Austrittsöffnung oder Spitze 7, die sich unter dem Druck der Injektionsflüssigkeit öffnet und nach abgeschlossener Injektion automatisch schließt.

Der Ohrkatheter 1 hat insgesamt eine Länge von etwa 15 cm bei einem Durchmesser des Katheterschlauchs 2 von 3 mm und einem Durchmesser des Injektionsschlauchs von etwa 1 mm.

Eine Hülse 8, die reibschlüssig auf dem Katheter aufsitzt, dient als Steuerungs- und Handhabungshilfe. Die Hülse ist vorzugsweise aus Kunststoff gefertigt und zweckmäßigerweise geschlitzt, um sie aufstecken oder abziehen zu können.

Bei der Behandlung eines Hörsturzes wird nach Anlage des Katheters beispielsweise über eine 2 ml Spritze Cortisonlösung in das Mittelohr gefüllt und dort für die erforderliche Zeit belassen. Die Füllung des Mittelohrs wird ohrmikroskopisch überwacht. Der Prozedur wird bevorzugt in örtlicher Betäubung durchgeführt, jedoch kann in besonderen Fällen eine Vollnarkose angeraten sein. Nach Applikation des Medikaments wird der proximale Teil des Applikationskatheters an der Wange mit einem Pflaster verklebt. Aus dem Injektionskanal wird der Druck abgelassen. Der Katheterballon verbleibt an Ort und Stelle in der Ohrtrompete und verhindert das Abfließen der injizierten Lösung. Nach Beendigung der Behandlung wird der Druck aus dem Ballon abgelassen und der Katheter gezogen.

## Patentansprüche

1. Nicht-invasiver Ohrkatheter zur Einführung in die Ohrtrompete mit einem Katheterschlauch (2), einem mit einem Druckmedium expandierbaren Ballon (3) zum Verschluss der Ohrtrompete und einem Injektionskanal (5) zur Einführung eines Medikaments in den Mittelohrbereich, wobei der Injektionskanal (5) ein druckabhängiges Einwegventil umfasst, **dadurch gekennzeichnet, dass** der Ohrkatheter eine Verschlussvorrichtung aufweist, die das applizierte Druckmedium nach Befüllen im Ballon (3) zurückhält und den expandierten Zustand des Ballons gewährleistet, und geeignet ist, das in das Mittelohr eingeführte Medikament über mehrere Tage im Mittelohr zurückzuhalten.

2. Ohrkatheter nach Anspruch 1, **dadurch gekennzeichnet, dass** der Injektionskanal (5) im Inneren des Katheters verläuft.

3. Ohrkatheter nach Anspruch 1 oder 2, **dadurch gekennzeichnet, dass** der expandierbare Ballon (3) als Low-Pressure-Cuff ausgebildet ist und entsprechend bei Expansion eine Manschette ausbildet, die die Ohrtrompete verschließt.

4. Ohrkatheter nach einem der Ansprüche 1 bis 3, **gekennzeichnet durch** einen Druckbegrenzer zur Begrenzung des Drucks des Ballons auf maximal 1 bar.

5. Ohrkatheter nach einem der vorstehenden Ansprüche, **dadurch gekennzeichnet, dass** der Injektionskanal (5) einen Luer-Lock-Anschluss (6) für eine Spritze mit Injektionsmaterial aufweist.

6. Ohrkatheter nach einem der vorstehenden Ansprüche, **dadurch gekennzeichnet, dass** der Katheterschlauch (2) einen Luer-Lock-Anschluss (4) für ein flüssiges Medium zur Expansion des Ballons (3) aufweist.

7. Ohrkatheter nach Anspruch 6, **dadurch gekennzeichnet, dass** das Druckmedium zur Expansion des Ballons (3) eine physiologische NaCl-Lösung ist.

8. Ohrkatheter nach einem der vorstehenden Ansprüche, **dadurch gekennzeichnet, dass** der Injektionskanal (5) distal vom Ballon (3) endet.

9. Ohrkatheter nach einem der vorstehenden Ansprüche, **dadurch gekennzeichnet, dass** der Injektionskanal (5) eine Austrittsöffnung (6) aufweist, die atraumatisch ausgebildet ist.

10. Ohrkatheter nach Anspruch 9, **dadurch gekennzeichnet, dass** die Austrittsöffnung (6) selbstschließend vorgesehen ist und als druckempfindliches Einwegventil dient.

11. Ohrkatheter nach einem der vorstehenden Ansprüche, **gekennzeichnet durch** eine Handhabungshilfe (8).

12. Ohrkatheter nach Anspruch 11, **dadurch gekennzeichnet, dass** die Handhabungshilfe (8) eine reibschlüssig auf dem Katheterschlauch aufsitzende Kunststoffhülse ist.

## Claims

1. Non-invasive ear catheter for introduction into the eustachian tube with a catheter tube (2), a balloon (3) expandable by a pressure medium for closing the eustachian tube, and an injection channel (5) for introducing a medication into the middle ear region, wherein the injection channel (5) comprises a pressure-dependent one-way valve, **characterized in that** the ear catheter has a closure device which retains the applied pressure medium in the balloon (3) after filling and ensures the expanded state of the balloon, and is suitable for retaining the medication in the middle ear for several days.

2. Ear catheter according to claim 1, **characterized in that** the injection channel (5) extends inside the catheter.

3. Ear catheter according to claim 1 or 2, **characterized in that** the expandable balloon (3) is designed as a low-pressure cuff and, when expanded, forms a cuff that closes the Eustachian tube.

4. Ear catheter according to any one of claims 1 to 3, **characterized by** a pressure limiter for limiting the pressure of the balloon to a maximum of 1 bar.

5. Ear catheter according to any one of the preceding claims, **characterized in that** the injection channel (5) is provided with a Luer lock connection (6) for a syringe with injection material.

6. Ear catheter according to any one of the preceding claims, **characterized in that** the catheter tube (2) is provided with a Luer lock connection (4) for a liquid medium for the expansion of the balloon (3).

7. Ear catheter according to claim 6, **characterized in that** the pressure medium for expanding the balloon (3) is a physiological NaCl solution.

8. Ear catheter according to any one of the preceding claims, **characterized in that** the injection channel (5) ends distally from the balloon (3).

9. Ear catheter according to any one of the preceding claims, **characterized in that** the injection channel (5) is provided with an outlet opening (6) that is of atraumatic design.

10. Ear catheter according to claim 9, **characterized in that** the outlet opening (6) is of self-closing design and serves as pressure sensitive one-way valve.

11. Ear catheter according to any one of the preceding claims, **characterized by** a handling aid (8).

12. Ear catheter according to claim 11, **characterized in that** the handling aid (8) is a plastic sleeve mounted friction-fitting on the catheter tube.

## Revendications

1. Cathéter auriculaire non invasif destiné à être introduit dans la trompe d'Eustache, comprenant un tube de cathéter (2), un ballon (3) pouvant être gonflé à l'aide d'un médium sous pression pour obturer la trompe d'Eustache et un canal d'injection (5) pour l'introduction d'un médicament dans la région de l'oreille moyenne, le canal d'injection (5) comprenant une valve unidirectionnelle dépendante de la pression, **caractérisé en ce que** le cathéter auriculaire comporte un dispositif de fermeture qui après remplissage du ballon (3), retient le médium sous pression injecte dans le ballon (3) et garantit l'état expansé du ballon, et est adapté pour retenir le médicament introduit dans l'oreille moyenne pendant plusieurs jours dans l'oreille moyenne.

2. Cathéter auriculaire selon la revendication 1, **caractérisé en ce que** le canal d'injection (5) s'étend à l'intérieur du cathéter.

3. Cathéter auriculaire selon la revendication 1 ou 2, **caractérisé en ce que** le ballon expansible (3) est conçu comme un manchon à basse pression et forme, lors de son expansion, un manchon qui ferme la trompe d'Eustache.

4. Cathéter auriculaire selon l'une des revendications 1 à 3, **caractérisé par** un limiteur de pression pour limiter la pression du ballon à 1 bar maximum.

5. Cathéter auriculaire selon l'une des revendications précédentes, **caractérisé en ce que** le canal d'injection (5) comporte un raccord Luer-Lock (6) pour une seringue contenant le produit à injecter.

6. Cathéter auriculaire selon l'une des revendications précédentes, **caractérisé en ce que** le tube du cathéter (2) comporte un raccord Luer-Lock (4) pour un medium fluide destiné à dilater le ballon (3).

7. Cathéter auriculaire selon la revendication 6, **caractérisé en ce que** le médium sous pression utilisé pour dilater le ballon (3) est une solution physiologique de NaCl.

8. Cathéter auriculaire selon l'une des revendications précédentes, **caractérisé en ce que** le canal d'injection (5) se termine distal du ballon (3).

9. Cathéter auriculaire selon l'une des revendications précédentes, **caractérisé en ce que** le canal d'injection (5) présente une ouverture de sortie (6) qui est conçue de manière atraumatique.

10. Cathéter auriculaire selon la revendication 9, **caractérisé en ce que** l'ouverture de sortie (6) est auto-obturante et sert de valve unidirectionnelle sensible à la pression.

11. Cathéter auriculaire selon l'une des revendications précédentes, **caractérisé par** un dispositif d'aide à la manipulation (8).

12. Cathéter auriculaire selon la revendication 11, **caractérisé en ce que** l'aide à la manipulation (8) est un manchon en plastique reposant par friction sur le tube du cathéter.
